(19) Europäisches Patentamt / European Patent Office / Office européen des brevets

(11) **EP 3 146 330 B1**

(12) **FASCICULE DE BREVET EUROPEEN**

(45) Date de publication et mention de la délivrance du brevet:
**24.07.2019 Bulletin 2019/30**

(21) Numéro de dépôt: **15727700.5**

(22) Date de dépôt: **12.05.2015**

(51) Int Cl.:
***G01N 33/18*** *(2006.01)*

(86) Numéro de dépôt international:
**PCT/FR2015/051246**

(87) Numéro de publication internationale:
**WO 2015/173510 (19.11.2015 Gazette 2015/46)**

(54) **PROCÉDÉ ET SYSTÈME SATELLITAIRE DE DÉTECTION DE NAPPE FLOTTANTE SUR UNE SURFACE MARINE À SURVEILLER**

SATELLITENVERFAHREN UND -SYSTEM ZUR DETEKTION EINER SCHWIMMSCHICHT AUF EINER ZU BEOBACHTENDEN MEERESOBERFLÄCHE

SATELLITE METHOD AND SYSTEM FOR DETECTING A FLOATING LAYER ON A SEA SURFACE TO BE MONITORED

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **14.05.2014 FR 1454267**

(43) Date de publication de la demande:
**29.03.2017 Bulletin 2017/13**

(73) Titulaire: **Electricité de France**
**75008 Paris (FR)**

(72) Inventeurs:
• **LENES, Arnaud**
**Londres SW11 5EL (GB)**
• **RITZ, Jean-Benoît**
**Londres SW6 6JX (GB)**

(74) Mandataire: **Cabinet Plasseraud**
**66, rue de la Chaussée d'Antin**
**75440 Paris Cedex 09 (FR)**

(56) Documents cités:
• **MASAHARU FUJITA ET AL: "SIR-B Experiments in Japan: Sensor Calibration and Oil Pollution Detection Over Ocean", IEEE TRANSACTIONS ON GEOSCIENCE AND REMOTE SENSING, IEEE SERVICE CENTER, PISCATAWAY, NJ, US, vol. 44, no. 4, 1 juillet 1986 (1986-07-01), pages 567-574, XP011159039, ISSN: 0196-2892**

• **PETER VOGT ET AL: "<title>Monitoring of marine oil spills from SAR satellite data</title>", PROCEEDINGS OF SPIE, vol. 5569, 16 novembre 2004 (2004-11-16), pages 209-219, XP055160861, ISSN: 0277-786X, DOI: 10.1117/12.565259**
• **BELOV E N ET AL: "APPLICATION OF GROUND-BASED AND AIR/SPACEBORNE RADARS FOR OIL SPILL DETECTION IN SEA AREAS", TELECOMMUNICATIONS AND RADIO ENGINEERING, SCRIPTA TECHNICA,INC., NEW YORK, NY, US, vol. 51, no. 1, 1 janvier 1997 (1997-01-01), pages 1-08, XP000834878, ISSN: 0040-2508**
• **Yahya A. Dehqanzada ET AL: "Secrets For Sale: How Commercial Satellite Imagery Will Change the World", , 1 janvier 2000 (2000-01-01), XP055160899, Extrait de l'Internet: URL:http://carnegieendowment.org/files/FIN ALreport.pdf [extrait le 2015-01-09]**
• **MERV FINGAS ET AL: "Review of oil spill remote sensing", MARINE POLLUTION BULLETIN, vol. 83, no. 1, 20 avril 2014 (2014-04-20) , pages 9-23, XP055160871, ISSN: 0025-326X, DOI: 10.1016/j.marpolbul.2014.03.059**
• **BREKKE C ET AL: "Oil spill detection by satellite remote sensing", REMOTE SENSING OF ENVIRONMENT, ELSEVIER, XX, vol. 95, no. 1, 15 mars 2005 (2005-03-15), pages 1-13, XP027794779, ISSN: 0034-4257 [extrait le 2005-03-15]**

- **ANNE H S SOLBERG ET AL: "Oil Spill Detection in Radarsat and Envisat SAR Images", IEEE TRANSACTIONS ON GEOSCIENCE AND REMOTE SENSING, IEEE SERVICE CENTER, PISCATAWAY, NJ, US, vol. 45, no. 3, 1 mars 2007 (2007-03-01), pages 746-755, XP011168376, ISSN: 0196-2892, DOI: 10.1109/TGRS.2006.887019**
- **Antunes Sónia: "OIL SPILL DETECTION USING SAR IMAGES", , 1 février 2011 (2011-02-01), XP055160940, Extrait de l'Internet: URL:http://www.lx.it.pt/~bioucas/files/PhD _sonia_oil_detection.pdf [extrait le 2015-01-09]**
- **LEE S MILLER ET AL: "Objectives and Capabilities of the Skylab S-193 Altimeter Experiment", IEEE TRANSACTIONS ON GEOSCIENCE ELECTRONICS, IEEE, US, vol. 1, no. 1, 1 janvier 1972 (1972-01-01) , pages 73-79, XP011147988, ISSN: 0018-9413**

**Description**

**Domaine technique**

L'invention concerne le domaine de la détection de nappe flottante à la surface de l'eau, notamment d'une surface marine à proximité du littoral. La détection de nappe flottante proposée vise en particulier à anticiper les risques et menaces inhérents à la présence de nappe flottante pour des installations en bord de mer telles qu'une centrale nucléaire.

**Etat de la technique**

Le système de refroidissement d'une centrale de production électrique installée sur le littoral (telle qu'une centrale thermique) est généralement alimenté par de l'eau en provenance de la mer. A cet effet, des tambours peuvent être utilisés pour filtrer l'alimentation en eau de mer des pompes du système de refroidissement.

Or, à la surface de la mer, des nappes flottantes d'espèces animales (banc de méduses par exemple) ou de pollution (nappe de pétrole ou de débris typiquement) peuvent se former. La présence de telles nappes flottantes peut venir encombrer les tambours de filtration. Au-delà d'un certain encombrement, le colmatage des tambours empêche les pompes de fonctionner normalement, risquant d'entraîner la perte de la source froide de la centrale.

En général, un arrêt du système de refroidissement est nécessaire pour désencombrer les tambours, impliquant simultanément un arrêt de la production électrique de la centrale.

Afin de prévenir les risques de colmatage des tambours et d'éviter les arrêts de production électriques qui en découlent, il existe un besoin d'anticiper les risques d'encombrement des tambours, notamment en détectant la présence de nappes flottantes à proximité de la centrale.

A ce jour, des campagnes de surveillance aérienne sont parfois mises en place pour surveiller les surfaces marines aux environs d'une centrale et pour détecter une éventuelle présence de nappes flottantes.

Masaharu Fujita et al.: "SIR-B Experiments in Japan: Sensor Calibration and Oil Pollution Détection Over Océan" (IEEE Transactions on Geoscience and Remote Sensing, vol. 44, no. 4, 1 juillet 1986, pages 567-574), Peter Vogt et al.: "Monitoring of marine oil spills from SAR satellite data" (Proceedings of SPIE, vol. 5569, 16 novembre 2004, pages 209-219), E. N. Belov et al.: "Application of Ground-Based And Air/Spaceorne Radars for Oil Spill Détection in Sea Areas" (Télécommunications and Radio Engineering, Scripta Technica Inc. , New York, vol. 51, no. 1, 1 janvier 1997, pages 1-08), Yahya A. Dehqanzada et al. : "Secrets For Sale: How Commercial Satellite Imagery Will Change the World", (1 janvier 2000, XP055160899, http:// carnegieendowment.org/files/FINALreport.pdf) et Merv Fingas et al.: "Review of oil spill remote sensing" (Marine Pollution Bulletin, vol. 83, no. 1, 20 avril 2014, pages 9-23) dévoilent des techniques antérieures de détection des nappes flottantes.

Toutefois, cette surveillance aérienne est limitée en temps et en espace, et doit être réalisée de jour. Par voie de conséquence, cette technique de surveillance est dans la pratique exclusivement utilisée pour la gestion de situations de crise déjà déclarées.

Il existe donc un besoin pour la mise en oeuvre d'une technique permettant une détection préventive des nappes flottantes, lesquelles sont potentiellement menaçantes pour un site d'intérêt tel qu'une centrale thermique.

Par ailleurs, les nappes flottantes ont un comportement à la surface de l'eau qui diffère en fonction de leur composition. Par exemple, une nappe d'espèce animale colmatante (type méduse ou algue) ne se comporte pas de la même manière qu'une nappe d'hydrocarbure et elles ne présentent pas le même risque de colmatage des tambours de la centrale.

Ainsi, il existe aussi un besoin de pouvoir caractériser la composition des nappes flottantes détectés à proximité de la centrale pour mieux anticiper les risques de colmatage.

L'invention vient améliorer la situation en ce sens.

**Résumé de l'invention**

L'objet de l'invention est de remédier aux inconvénients précités et de répondre aux besoins énoncés en utilisant notamment des mesures radars satellitaires pour détecter les nappes à la surface de l'eau et, à partir des mesures satellitaires, interpréter les propriétés fluidiques des nappes détectées pour en déterminer la composition.

A cet effet, un premier aspect de l'invention concerne un procédé de détection de nappe flottante sur une zone de surveillance de surface marine, un site d'intérêt étant placé dans ou en périphérie de la zone de surveillance. Le procédé comprend au moins les étapes de :

    a) mesure satellitaire d'un retour de signal radar, le signal radar étant émis par un satellite vers la surface marine de la zone de surveillance ;

    b) reconnaissance d'au moins un profil de houle de la surface marine en fonction des mesures satellitaires ;

    c) identification des propriétés fluidiques correspondant aux profils de houle reconnus ; et

    d) émission d'une alerte lorsque les propriétés fluidiques identifiées pour un des profils reconnus correspondent à une surface marine comportant des éléments parasites pour le site d'intérêt.

Il convient de comprendre par « en périphérie » une localisation du site d'intérêt pouvant être située dans un espace s'étendant autour de la zone de surveillance, par exemple d'une distance allant jusqu'à une centaine de kilomètres.

L'utilisation de mesures satellitaires permet de couvrir de grands espaces de façon presque permanente grâce au survol régulier de la zone de surveillance par des satellites.

En outre, les mesures radars utilisées permettent de s'affranchir de la présence de couverture nuageuse au-dessus de la zone de surveillance et peuvent être réalisées de jour comme de nuit (les nuages et les conditions de luminosité n'impactant pas les niveaux de détection des mesures radars satellitaires).

Les mesures satellitaires permettent de déterminer des variations :

- altimétriques de la surface de la mer selon la longueur d'onde et l'amplitude des niveaux mesurés de retour de signal et/ou

- de rugosité de la surface de la mer en fonction des valeurs moyennes des niveaux mesurés du retour de signal.

Il convient de comprendre par « rugosité » de la surface marine, une très légère variation de relief à la surface de la mer, pouvant se manifester sous la forme de vaguelettes ou de rides d'eau.

Ces variations altimétriques et de rugosité permettent de discriminer des caractéristiques de houle de la surface marine. Les parties de la zone de surveillance qui présentent des caractéristiques de houle similaires correspondent à un profil de houle de cette zone. Typiquement, des parties de la zone de surveillance présentant de fortes variations altimétriques peuvent correspondre à un premier profil alors qu'une partie de la zone de surveillance ayant de faibles variations altimétriques peuvent correspondre à un deuxième profil. A partir des profils de houle identifiés sur la zone de surveillance, une nappe flottante peut être reconnue pour une zone ayant un profil de houle différent de la houle environnante.

Les mesures satellitaires peuvent en outre être interprétées pour déterminer les propriétés fluidiques de la surface marine. En l'occurrence, les propriétés fluidiques de la surface marine peuvent être évaluées à partir du comportement de la houle à la surface de l'eau, i.e. selon les caractéristiques de houle du ou des profils de houle identifiés sur la zone de surveillance. Les propriétés fluidiques déterminées permettent de qualifier la composition de la surface de l'eau et d'estimer la concentration d'éléments parasites pour le site d'intérêt.

Lorsque les caractéristiques de houle d'un profil de houle identifié correspondent à une surface marine comprenant des éléments parasites pour le site d'intérêt, l'émission d'une alerte permet de prévenir d'un risque d'encombrement de tambour de filtrage par exemple. Ainsi, il est possible de mettre en place des mesures préventives pour éviter un colmatage.

Typiquement, le site d'intérêt peut être une centrale thermique telle qu'une centrale nucléaire installée sur le littoral, une plateforme pétrolière, une usine de dessalement, une ferme aquacole, ou autre.

Les éléments parasites pour le site d'intérêt peuvent se manifester sous forme :

- d'espèces marines telles que des méduses, cténaires, algues ou poissons ;

- de pollution comme des hydrocarbures ou des débris ;

- ou autre.

Avantageusement, la zone de surveillance est dimensionnée en fonction de paramètres parmi :

- des vitesses de courants marins ;
- un coefficient de marée ;
- des conditions météorologiques ;
- une fréquence de surveillance prédéterminée ; et
- une fréquence de survol de la zone de surveillance par le satellite.

A titre d'exemple, la taille de la zone de surveillance peut être :

- grande (entre 10 et 100 km2) si la fréquence de survol de la zone de surveillance est faible (une fois par jour typiquement) et la vitesse des vents élevés, et
- petite (entre 1 et 10 km2) si la fréquence de survol de la zone de surveillance est élevée (tous les quarts d'heure par exemple) et la vitesse des vents faibles.

Ainsi, la zone de surveillance peut être ajustée selon les conditions dans lesquelles se déroule la surveillance, permettant de limiter d'éventuelles perturbations des mesures inhérentes aux conditions susmentionnées. A titre purement qualitatif, les dimensions de la zone de surveillance sont ajustées en fonction des conditions météorologiques. La qualité et la précision de détection de nappe flottante peuvent ainsi être encore améliorées.

Selon l'invention à l'étape b), des limites singulières de profil de houle peuvent en outre être reconnues sur la zone de surveillance, les limites singulières correspondant à de fortes variations localisées des niveaux de mesures satellitaires.

Les limites singulières identifiées délimitent des zones où les caractéristiques de houle sont fortement modifiées, ce qui est généralement constaté à la transition entre deux profils de houle différents. Des modifications brutales des caractéristiques de houle traduisent un changement des propriétés du fluide (ou des caractéristiques bathymétriques), et donc potentiellement un changement local de la composition de la surface marine. Les limites singulières reconnues permettent donc de délimiter plus nettement les profils de houle sur la zone de surveillance.

En complément, les limites singulières reconnues peuvent être comparées à une cartographie bathymétrique relative à la zone de surveillance.

De cette manière, il est possible d'identifier les modifications de la houle causées par la réfraction bathymétrique et de les isoler pour ne pas qu'elles biaisent la reconnaissance de limites singulières de profils de houle. Un changement brutal des caractéristiques de la houle qui n'est pas localisé à une zone de fort changement de la bathymétrie, ne peut être associé à la réfraction bathymétrique. Ce changement peut dès lors être associé à un changement de composition de la surface marine, traduisant la présence potentielle d'une nappe flottante.

Ainsi, les éventuelles erreurs de détection de nappes causées par les hauts fonds marins sont évitées.

Selon un premier mode de réalisation avantageux, l'au moins un profil de houle reconnu correspond à un ensemble de mesures sensiblement de même niveau, l'ensemble de mesures étant délimité au moins en partie par les limites singulières reconnues.

Il convient de comprendre par « sensiblement de même niveau » un ensemble de mesures de même niveau ou variant autour d'une même valeur moyenne de niveau de détection.

Des mesures sensiblement de même niveau peuvent notamment correspondre à une surface d'eau de même rugosité. En outre, lorsque l'ensemble de mesures est délimité au moins en partie par les limites singulières reconnues, l'ensemble de mesures peut correspondre à un profil de houle caractérisé par cette même rugosité.

A titre d'exemple purement illustratif, une surface marine en présence d'une nappe d'hydrocarbures est généralement plus lisse (moins rugueuse) qu'à la surface de l'eau environnante. La nappe d'hydrocarbures est le plus souvent entourée de variations brutales de la houle à sa périphérie, pouvant se manifester via une atténuation rapide de la houle environnante en périphérie de la nappe ou via des amplitudes de houle se démarquant des profils identifiés sur la zone de surveillance.

Avantageusement, sur la zone de surveillance, peuvent être reconnus au moins :

- un premier profil de houle selon un ensemble de mesures sensiblement d'un même premier niveau, et

- un deuxième profil de houle selon un ensemble de mesures sensiblement d'un même deuxième niveau.

Les premier et deuxième niveaux peuvent être comparés. Le premier ou le deuxième profil de houle correspondant au plus faible des niveaux comparés peut être associé à des propriétés fluidiques de surface marine comportant des éléments parasites pour le site d'intérêt.

Selon ce mode réalisation, les premier et deuxième niveaux correspondent à des rugosités (i.e. vaguelettes surfaciques de la houle) différentes de la surface marine. En effet, les zones de forte rugosité renvoient un écho radar fort vers le satellite et, à l'inverse, les zones de faible rugosité réfléchissent très peu le signal radar vers le satellite (phénomène de réflexion dans une direction ayant un angle par rapport à la surface marine qui est équivalent à l'angle d'incidence du signal radar envoyé).

Des parasites tels que des nappes d'hydrocarbures peuvent modifier la rugosité de la surface marine. Typiquement, en présence d'hydrocarbures, la surface marine se compose de deux fluides non miscibles dont le plus léger (i.e. les hydrocarbures) surnage. La nappe d'hydrocarbures formée en surface a des effets sur la viscosité et la tension de surface qui sont très marqués et impactent fortement les ondes de faible longueur d'onde et de faible amplitude, modifiant ainsi la rugosité apparente à la surface de la mer. Des changements singuliers de rugosité apparaissent dès lors à la surface de l'eau entre :

- la nappe d'hydrocarbures qui est de rugosité faible et

- la surface de la mer environnante qui est de rugosité plus élevée.

Le plus faible des premier et deuxième niveaux mesurés par le satellite peut donc correspondre à une surface marine pouvant comporter des éléments parasites pour le site d'intérêt tels qu'une nappe d'hydrocarbures.

Selon un deuxième mode de réalisation avantageux, l'au moins un profil de houle reconnu correspond à un ensemble de mesures dont le niveau varie selon une même longueur d'onde et une même amplitude, l'ensemble de mesures étant délimité au moins en partie par les limites singulières reconnues.

Un ensemble de mesures délimité par les limites singulières reconnues et dont les niveaux des mesures varient selon une même longueur d'onde et une même amplitude, peuvent caractériser un profil de houle. Une surface marine se comporte généralement de manière uniforme sur des zones exposées aux mêmes phénomènes météorologiques avec notamment une même longueur d'onde et une même amplitude de houle.

Selon l'invention à l'étape c), les propriétés fluidiques de l'au moins un profil de houle reconnu sont identifiées par :

- détermination d'une fraction volumique en fonction de :

○ un coefficient d'atténuation de l'amplitude de la houle au niveau des limites singulières,

○ un nombre d'ondes au niveau des limites singulières déterminé à partir de la longueur d'onde du profil de houle reconnu,

- identification de la fraction la plus proche de la fraction volumique déterminée parmi des fractions volumiques prédéfinies, les fractions volumiques prédéfinies étant associées à des propriétés fluidiques prédéterminées ; et

- association au profil de houle reconnu des propriétés fluidiques prédéterminées de la fraction volumique prédéfinie identifiée comme la plus proche de la fraction volumique déterminée.

Les vagues en mer sont atténuées lorsqu'elles rencontrent des nappes d'éléments parasites tels que des bancs d'espèces colmatantes (méduses, algues ou autre) et le comportement d'atténuation permet de déterminer une fraction volumique de la surface marine qui dépend de l'atténuation des vagues au niveau du profil de houle détecté.

La fraction volumique déterminée peut être comparée à une base de données de fractions volumiques. Les fractions volumiques stockées dans la base de données peuvent notamment être des fractions prédéterminées pour différentes espèces colmatantes et différentes conditions climatiques et météorologiques. Les espèces colmatantes répertoriées dans cette base de données peuvent notamment être renseignées selon différentes fractions volumiques traduisant les propriétés fluidiques qu'elles induisent à la surface de l'eau, et notamment leur comportement en fonction des saisons, des conditions météorologiques, des conditions de luminosité, de température de l'eau ou autre, de sorte à pouvoir les reconnaitre en fonction des conditions dans lesquelles sont effectuées les mesures.

Par exemple, selon si les mesures sont réalisées en diurne ou en nocturne, une espèce comatante de type méduse ne se comporte pas de la même manière. De telles variations de comportement ont un impact sur la houle et peuvent être identifiées par mise en correspondance de la fraction issue des mesures avec celles de la base de données.

En outre, les fractions volumiques prédéfinies peuvent correspondre à des fractions volumiques constatées, dans des conditions météorologiques connues, pour des surfaces maritimes comportant des corps flottants identifiés.

Ainsi, une base de données de fractions volumiques peut être constituée au préalable pour déterminer par la suite quels sont les éléments parasites détectés sur la zone de surveillance.

En complément, la fraction volumique déterminée pour un profil de houle peut en outre être sensible aux effets de la tension superficielle de la surface marine correspondante.

Avantageusement, le procédé comprend en outre une étape de détermination de déplacement du profil de houle correspondant à une surface marine comportant des éléments parasites pour le site d'intérêt, la détermination de déplacement étant fonction de modèles hydrodynamiques préétablis de la zone de surveillance ou de modèles hydrodynamiques issus de sources d'information sur les conditions hydrodynamiques de la zone de surveillance.

Ainsi, il est possible de prévoir le déplacement d'une nappe flottante menaçante pour le site d'intérêt et d'anticiper les mesures à mettre en place pour protéger par exemple le circuit de refroidissement d'une centrale thermique. La gestion et la protection de la source froide d'une telle centrale sont dès lors améliorées, permettant de prévenir les risques de colmatage des tambours et d'éviter les arrêts de production électriques qui en découlent.

Selon un deuxième aspect, l'invention propose un système de détection de nappe flottante sur une zone de surveillance de surface marine, un site d'intérêt étant placé dans ou en périphérie de la zone de surveillance.

Le système comprend au moins :

- un satellite configuré pour :

○ émettre un signal radar vers la surface marine de la zone de surveillance ;

○ mesurer un retour du signal radar émis ;

○ communiquer des données relatives aux niveaux de mesure des retours de signal mesurés ;

- une unité de gestion comportant :

○ une interface de communication configurée pour recevoir les données communiquées par le satellite, et

○ une unité de traitement de données configurée pour mettre en oeuvre les étapes du procédé de détection selon le procédé susmentionné.

Par ailleurs, le système peut comprendre une base de données configurée pour stocker des données relatives à :

- des fractions volumiques prédéterminées correspondant à des fractions volumiques constatées, dans des conditions météorologiques connues, pour des surfaces maritimes comportant des corps flottants identifiés ; et/ou

- une cartographie bathymétrique relative à la zone de surveillance.

En outre, le site d'intérêt peut être une installation de production électrique telle qu'une centrale nucléaire ou des hydroliennes.

La présente invention vise également un programme informatique comportant des instructions pour la mise en oeuvre du procédé précédemment décrit, lorsque ce programme est exécuté par une unité de traitement de données telle qu'un processeur.

Ce programme peut utiliser n'importe quel langage de programmation (par exemple, un langage objet ou autre), et être sous la forme d'un code source interprétable, d'un code partiellement compilé ou d'un code totalement compilé.

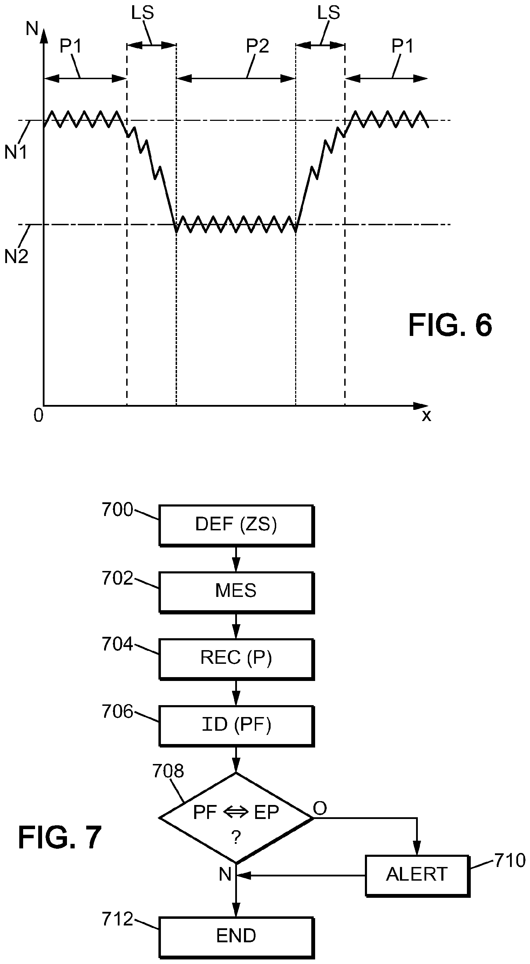

La figure 7 décrite en détails ci-après, peut former un organigramme de l'algorithme général d'un tel programme informatique.

**Brève description des figures**

D'autres caractéristiques et avantages de l'invention apparaîtront à l'examen de la description détaillée ci-après, et des figures annexées sur lesquelles :

- les **figures 1a et 1b** illustrent un exemple de système de détection de nappe flottante selon l'invention ;

- la **figure 2** illustre un exemple d'image satellite d'un littoral où est installé une centrale thermique, une nappe flottante étant à proximité de la centrale ;

- la **figure 3** est un exemple de vue en coupe de la houle à la surface de la mer au niveau de la nappe flottante ;

- la **figure 4** est un exemple de représentation graphique des niveaux des mesures effectuées par le satellite au niveau de la nappe flottante située dans la zone à surveiller ;

- la **figure 5** est un exemple d'illustration d'une nappe flottante d'hydrocarbures vue du ciel ;

- la **figure 6** est un exemple de représentation graphique des niveaux de mesures effectuées par le satellite au niveau de la nappe flottante d'hydrocarbures située dans la zone à surveiller ; et

- la **figure 7** est un organigramme composé d'un exemple de succession d'étapes du procédé de détection selon l'invention.

Pour des raisons de clarté, les dimensions des différents éléments représentés sur ces figures ne sont pas en proportion avec leurs dimensions réelles. Sur les figures, des références identiques correspondent à des éléments identiques pour les différents modes de réalisation exposés.

**Description détaillée**

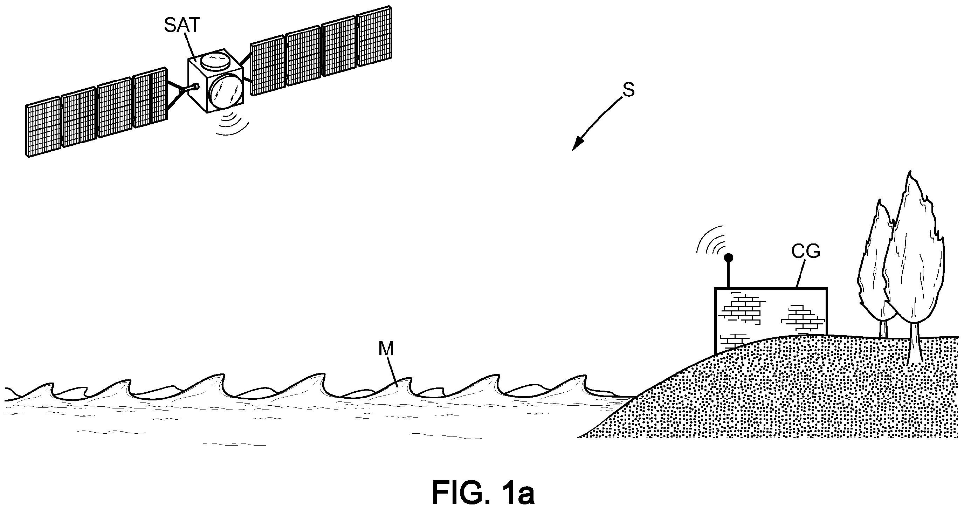

On se réfère tout d'abord à la **figure 1a** qui illustre un exemple de réalisation du système de détection de nappe flottante selon l'invention.

Dans cet exemple, le système S comporte un satellite SAT configuré pour :

◦ émettre un signal radar vers la mer M, et plus particulièrement vers une surface marine à surveiller ;

◦ mesurer un retour du signal radar émis ;

◦ communiquer des données relatives aux niveaux de mesure des retours de signal mesurés.

A cet effet, le satellite SAT peut nomment comprendre un émetteur radar altimétrique fonctionnant dans une gamme de fréquence non visible, émettant par exemple des signaux selon une fréquence comprise entre 12 et 18 GHz. La précision des mesures altimétriques est préférentiellement de 10 centimètres verticalement et de 1 mètre horizontalement afin de pouvoir discerner correctement les différentes houles à la surface de l'eau.

En outre, le satellite SAT peut comprendre un module de communication pour envoyer à un centre de gestion CG les données relatives aux mesures effectuées. Le centre CG traite les données envoyées par le satellite pour détecter les nappes flottantes à la surface de la mer M.

Dans cet exemple de réalisation, le centre CG est une entité séparée. Toutefois, selon d'autre modes de réalisation possible, le centre CG peut être embarqué directement dans le satellite ou installé au sein même d'une centrale thermique.

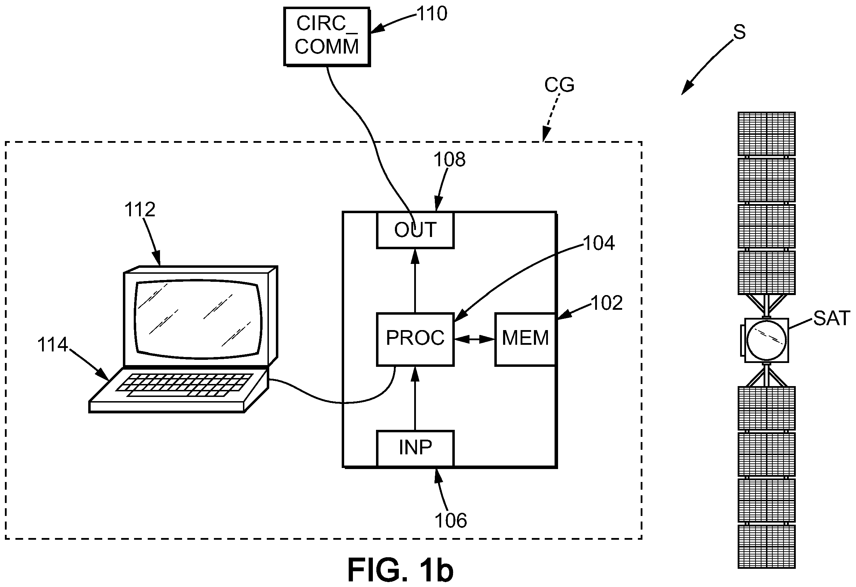

On se réfère tout d'abord à la **figure 1b** qui représente le centre de gestion CG du système S. Le centre CG peut être un ordinateur comprenant une mémoire 102 pour stocker :

- des instructions permettant la mise en oeuvre du procédé,

- des données satellitaires reçues, et

- des données temporaires pour réaliser les différentes étapes du procédé tel que décrit précédemment et aussi détaillé plus loin.

La mémoire MEM peut en outre stocker une base de données comportant :

- des fractions volumiques prédéfinies correspondant à des fractions volumiques constatées, dans des conditions météorologiques connues, pour des surfaces maritimes comportant des corps flottants identifiés ; et/ou

- une cartographie bathymétrique relative à la zone à surveiller.

L'ordinateur comporte en outre une unité de traitement de données 104. Cette unité de traitement de données peut être un circuit comme par exemple :

- un processeur apte à interpréter des instructions sous la forme de programme informatique, ou

- une carte électronique dont les étapes du procédé de l'invention sont décrites dans le silicium, ou encore

- une puce électronique programmable comme une puce FPGA (pour « Field-Programmable Gâte Array » en anglais).

Cet ordinateur comporte une interface d'entrée 106 pour la réception de données satellitaires, et une interface de sortie 108 pour la fourniture à un dispositif distant 110 d'alertes en cas de détection d'une nappe flottante. Enfin, l'ordinateur peut comporter, pour permettre une interaction aisée avec un utilisateur, un écran 112 et un clavier 114. Bien entendu, le clavier est facultatif, notamment dans le cadre d'un ordinateur ayant la forme d'une tablette tactile, par exemple.

L'interface d'entrée 106 peut notamment recevoir des modèles hydrodynamiques issus de sources d'information sur les conditions hydrodynamiques de la zone à surveiller telles qu'un serveur de centre de surveillance maritime.

Typiquement, le dispositif distant 110 peut être une plateforme de supervision de centrale de production électrique ou un terminal de client apte à recevoir les alertes du centre CG via l'interface 108. A cet effet, le dispositif 110 peut comporter une interface de communication apte à recevoir les données communiquées par le centre CG et une unité de traitement de données pour les interpréter. Ainsi, les alertes reçues par le dispositif distant 110 peuvent être utilisées par ce dernier pour anticiper de potentiels encombrements et/ou colmatages. A titre d'exemple purement illustratif, un utilisateur peut recevoir sur son téléphone les alertes concernant une centrale électrique qu'il supervise. Ainsi, l'utilisateur peut mettre en place des actions préventives permettant d'anticiper les risques de colmatage (et ainsi les besoins de couper la production d'énergie électrique de la centrale). Des services variés peuvent être en outre proposés aux utilisateurs en fonction de différentes interprétations possibles des alertes par le dispositif distant 110.

De surcroît, l'interface 106 peut recevoir des données qualitatives et quantitatives relatives aux conditions météorologiques et aux vents. Ces données peuvent par exemple provenir d'un anémomètre placé au niveau ou à proximité du centre de gestion CG, ou tout au moins à proximité de la zone de mer à surveiller et de la centrale thermique. Ces données peuvent notamment être prises en compte pour affiner la détermination de fraction volumique correspondant aux mesures altimétriques envoyées par le satellite.

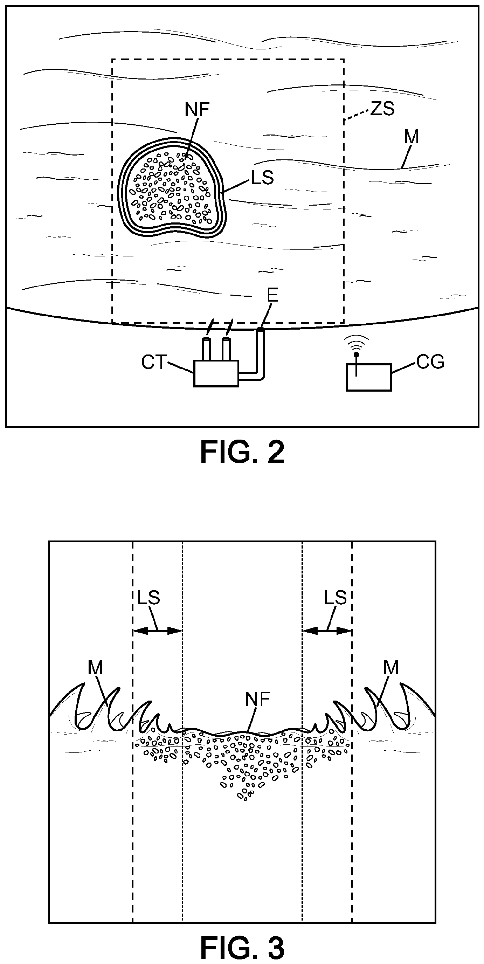

A la **figure 2,** il est illustré un exemple d'image satellite d'un littoral où est installée une centrale thermique CT. La zone surveillée par le satellite est la zone de surveillance ZS, située à proximité de l'entrée E du système de refroidissement de la centrale CT.

Par l'intermédiaire des mesures satellitaires reçues par le centre de gestion CG, il est possible de détecter les variations altimétriques à la surface de la mer M. Sur la mer M, une nappe flottante NF est actuellement présente. Dans cet exemple, la nappe NF est un banc de méduses.

En périphérie de la nappe NF, la houle de la mer M est modifiée brutalement. Les variations rapides de la houle sur cette périphérie forment les limites singulières LS.

A la **figure 3,** on a représenté un exemple de vue en coupe de la houle à la surface de la mer M au niveau de la nappe flottante NF. La houle en mer M suit une longueur d'onde et une amplitude régulières. La houle de la nappe NF est différente à cause de la présence des corps flottants que sont les méduses. Dans cet exemple, la houle de la nappe NF est quasi-nulle. La houle de la mer est rapidement atténuée en périphérie de la nappe NF, formant des limites singulières qui séparent le profil de houle de la mer du profil de houle de la nappe.

Les limites singulières LS sont les zones à la périphérie des nappes NF pour lesquelles on observe une atténuation plus marquée de la houle. Cette singularité est causée par une viscosité locale plus importante du fluide liée a la présence de corps flottants (e.g. méduses ou autre).

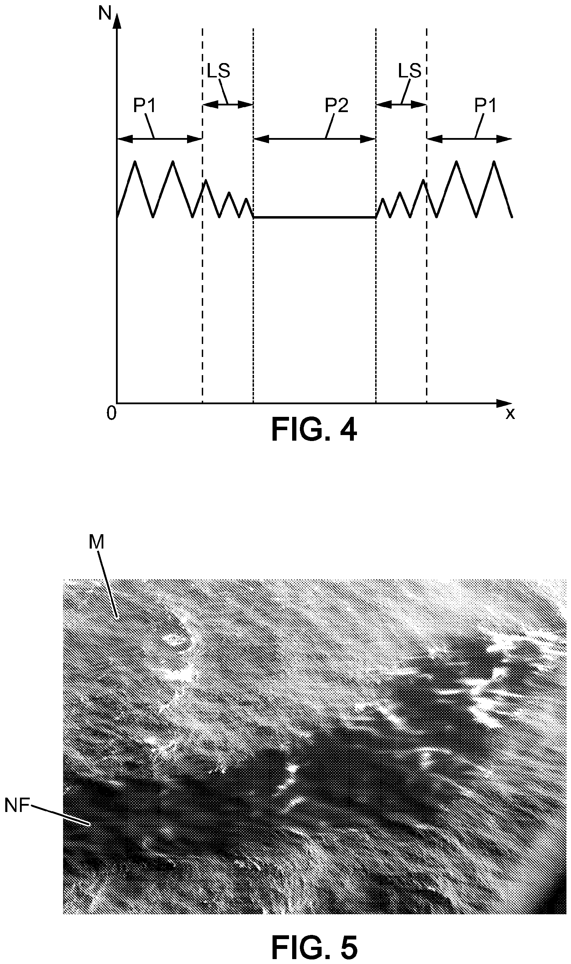

A la **figure 4,** on a représenté sur un graphique dans lequel apparait la variation du niveau de détection N des mesures en fonction de la position x des mesures sur la zone de surveillance ZS. Les mesures satellitaires du retour de signal radar ont des variations de niveaux de détection correspondant aux variations altimétriques à la surface de l'eau.

A partir des mesures satellitaires, le centre de gestion CG peut reconnaître :

- un premier profil de houle et un deuxième profil de houle pour les ensembles de mesures P1 et P2 ayant des niveaux correspondant respectivement aux mêmes caractéristiques de houle (même longueur d'onde et même amplitude) ; et

- les limites singulières LS qui correspondent aux fortes variations localisées des niveaux de mesures satellitaires.

A partir des mesures effectuées, une cartographie des caractéristiques mécaniques de la houle est obtenue pour la zone de surveillance ZS.

On se réfère maintenant à la **figure 5** sur laquelle est illustré une nappe flottante NF d'hydrocarbures.

La nappe d'hydrocarbures formée en surface de la mer M a des effets sur la viscosité et la tension de surface modifiant ainsi la rugosité apparente à la surface de l'eau.

La nappe NF est d'une rugosité moindre que la rugosité de la mer. Aussi, les profils de houle peuvent être discriminés par l'intermédiaire de leurs rugosités respectives qui diffèrent.

A la **figure 6,** on a représenté sur un graphique dans lequel apparaît également, tout comme la figure 4, la variation du niveau de détection N des mesures en fonction de la position x des mesures sur la zone de surveillance ZS.

Les variations de niveaux de mesures oscillent entre un premier niveau N1 et un deuxième niveau N2 selon la localisation des mesures sur la zone ZS. Les premier et deuxième niveaux correspondent à des rugosités (i.e. vaguelettes surfaciques de la houle) différentes de la surface marine. Comme susmentionné, les zones de forte rugosité renvoient un écho radar fort vers le satellite et, à l'inverse, les zones de faible rugosité réfléchissent très peu le signal radar vers le satellite.

Les profils de houle de la mer M et de la nappe NF peuvent ainsi être reconnus via :

- un premier ensemble de mesures P1 sensiblement du premier niveau N1, correspondant au profil de houle de la mer M, et

- un deuxième ensemble de mesures P2 sensiblement du deuxième niveau N2, correspondant au profil de houle de nappe NF.

On se réfère maintenant à la **figure 7** sur laquelle est illustré un organigramme composé d'un exemple de succession d'étapes du procédé de détection de nappe flottante.

A l'étape 700, les dimensions et la localisation de la zone de surveillance ZS sont définies (DEF(ZS)). La zone de surveillance peut être dimensionnée en fonction de paramètres parmi :

- des vitesses de courants marins ;

- un coefficient de marée ;

- des conditions météorologiques ;

- une fréquence de surveillance souhaitée ; et

- une fréquence de survol de la zone de surveillance par le satellite.

La zone de surveillance est localisée au-dessus d'un site d'intérêt tel que la centrale CT ou tout au moins en périphérie de ce site (par exemple en mer, au large de la centrale CT).

Selon une étape 702, le satellite SAT émet des signaux radars vers la surface marine de la zone de surveillance ZS et mesure le niveau du retour de signal (MES). Les mesures satellitaires de type radar permettent d'obtenir une cartographie spatiale et instantanée de l'état de surface de la mer (géométrie des vagues). L'information brute obtenue du satellite est l'altimétrie de la surface marine de la zone de surveillance. La résolution de cette cartographie est liée directement aux performances du satellite. Le choix des satellites utilisés peut prendre en compte comme paramètre la résolution souhaitée pour l'analyse des caractéristiques de houle (amplitude et longueur d'onde des vagues).

Selon une étape 704, le centre de gestion CG reçoit les données satellitaires relatives aux mesures et les interprète de sorte à reconnaître au moins un profil P de houle de la surface marine (REC(P)). Un profil de houle peut être reconnu pour un ensemble de mesures correspondant à des caractéristiques de houle similaires.

Pour mieux distinguer les différents profils de houle présents, les zones de transition rapides sont identifiées sur la zone de surveillance. Ces zones de transition rapide forment les limites singulières LS de profil de houle et correspondent à de fortes variations localisées des niveaux de mesures satellitaires. Les limites singulières identifiées délimitent la transition entre deux profils de houle différents.

En outre, pour s'assurer que les zones des transitions rapides ne sont pas inhérentes à des hauts fonds sur la zone de surveillance, les limites singulières reconnues peuvent être comparées à une cartographie bathymétrique de la zone de surveillance. Un recoupement avec la cartographie de la bathymétrie permet d'identifier et d'isoler les modifications de la houle causées par la réfraction bathymétrique.

Cette étape d'identification des limites singulières de changement de la houle peut être réalisée sur la base d'un examen visuel d'un opérateur à partir de la cartographie altimétrique obtenue selon les mesures satellitaires pour la zone de surveillance. Cette étape peut aussi être réalisée par un traitement automatique identifiant les limites singulières sur la base d'un critère de dépassement du gradient spatial des caractéristiques mécaniques de la houle.

La reconnaissance de profil de houle peut être réalisée par identification :

- d'un ensemble de mesures sensiblement de même niveau, l'ensemble de mesures étant délimité au moins en partie par les limites singulières reconnues, ou

- d'un ensemble de mesures dont le niveau varie selon une même longueur d'onde et une même amplitude, l'ensemble de mesures étant délimité au moins en partie par les limites singulières reconnues.

Selon une étape 706, sont identifiées (ID(PF)) les propriétés fluidiques PF correspondant aux profils de houle.

Lorsqu'au moins deux profils de houle correspondent respectivement à des ensembles de mesures sensiblement de mêmes niveaux, les premier et deuxième niveaux peuvent être comparés. Typiquement, selon l'exemple de la figure 6, le premier profil de houle (correspondant à l'ensemble de mesures P1) est identifié selon l'ensemble de mesures sensiblement du premier niveau N1, et le deuxième profil de houle (correspondant à l'ensemble de mesures P2) est identifié selon l'ensemble de mesures sensiblement du deuxième niveau N2.

En fonction de la comparaison des niveaux, le premier ou le deuxième profil de houle correspondant au plus faible des niveaux comparés peut être associé à des propriétés fluidiques PF de surface marine comportant des éléments parasites pour le site d'intérêt. L'autre des premier et deuxième profils (correspondant au plus élevé des niveaux) peut être associé quant à lui à des propriétés fluidiques classiquement observées pour une surface marine en pleine mer.

Lorsque le profil de houle correspond à un ensemble de mesures dont le niveau varie selon une même longueur d'onde et une même amplitude, les propriétés fluidiques de l'au moins un profil de houle reconnu sont identifiées par :

- détermination d'une fraction volumique en fonction de :

  ∘ un coefficient d'atténuation $\alpha$ de l'amplitude de la houle au niveau des limites singulières,

  ∘ un nombre d'ondes k au niveau des limites singulières déterminé à partir de la longueur d'onde du profil de houle reconnu,

- identification de la fraction la plus proche de la fraction volumique déterminée parmi des fractions volumiques prédéfinies, les fractions volumiques prédéfinies étant associées à des propriétés fluidiques prédéterminées ; et

- association au profil de houle reconnu des propriétés fluidiques prédéterminées de la fraction volumique prédéfinie identifiée comme la plus proche de la fraction volumique déterminée.

L'approche consiste à relier les variations des caractéristiques mécaniques de la houle (atténuation de la houle) aux propriétés fluidiques de la surface marine et peut utiliser à cet effet les équations de mécanique des fluides qui se basent sur :

- les lois de conservation de l'énergie (sous la forme de la relation entre l'atténuation des vagues au niveau des limites singulières et la viscosité de fluide),

- une relation empirique, pour les mélanges en suspension, entre la viscosité apparente et la concentration de particules solides.

En ce qui concerne les lois de conservation de l'énergie, l'atténuation $\alpha$ de la houle par dissipation visqueuse peut être déterminée via la loi exponentielle suivante :

$$A = A_0\, e^{-\alpha x}$$

Cette relation permet de calculer l'atténuation $\alpha$ des vagues en fonction de l'amplitude des vagues détectées pour le profil de houle reconnu, une amplitude de référence $A_0$ et la largeur x des limites singulières LS.

Le coefficient d'atténuation $\alpha$ est ainsi obtenu de façon pratique en mesurant graphiquement l'atténuation de l'amplitude sur la cartographie des mesures altimétriques.

A partir de l'atténuation $\alpha$ calculée au niveau des limites séparatives, la viscosité $\mu$ du profil de houle peut être calculée selon une relation pour les ondes de gravité du type :

$$\mu = \rho\,\omega\,\alpha\,(4k^3)^{-1}$$

Dans cette relation, k est le nombre de houle k détecté au niveau des limites singulières LS, $\alpha$ correspondant à l'atténuation calculée selon la relation ci-avant, $\rho$ étant la densité du fluide et $\omega$ correspondant à la pulsation de la houle.

Pour certains régimes de houle (notamment de faible amplitude et de faible longueur d'onde), les effets de tension superficielle ne sont pas négligeables et doivent être pris en compte. Selon une autre réalisation possible, la viscosité $\mu$ peut alors être calculée en fonction des effets de tension de surface $\sigma$, et ce de la manière suivante :

$$\mu = \left( \frac{\alpha\rho}{2k^2} \right) \left( \frac{g + 3\dfrac{\sigma k^2}{\rho}}{2(kg + \dfrac{\sigma k^3}{\rho})^{1/2}} \right)$$

La viscosité $\mu$ de la surface marine correspondant au profil de houle peut permettre de caractériser la viscosité du mélange en suspension à la surface de l'eau de mer.

A cet effet, on se place dans le cas d'un mélange en suspension avec une densité considérée comme constante entre le fluide (eau de mer) et les particules solides (espèces marines). La viscosité d'un tel mélange en suspension (eau de mer/espèce marines) peut être

défini par :

$$\mu_s = \mu_r * \mu_l$$

Dans cette relation, $\mu_s$ correspond à la viscosité du mélange, $\mu_l$ correspond à la viscosité de l'eau de mer et la $\mu_r$ est une viscosité relative (sans dimension).

Il existe plusieurs modèles empiriques pour définir $\mu_r$ en fonction de la fraction volumique $\varphi$ des particules solides. Par exemple, le modèle empirique de Thomas est le suivant :

$$\mu_r = 1 + 2{,}5\ \varphi + 10{,}05\ \varphi^2 + A\ e^{\beta\varphi},$$

avec A = 0,00273 et $\beta$ = 16,6.

Il existe également le modèle Kitano dans lequel :

$$\mu_r = (1 - \left(\frac{\phi}{A}\right))^{-2},$$

avec A = 0,68 pour les particules sphériques.

D'autres modèles similaires peuvent aussi être utilisés tels que le modèle empirique de Krieger et Dougherty.

A partir de la viscosité relative $\mu_r$, on peut donc déterminer la fraction volumique $\varphi$ du mélange au niveau de la surface marine ayant le profil de houle reconnu. Cette fraction volumique qualifie les propriétés fluidiques du profil reconnu.

De surcroît, des données qualitatives et quantitatives relatives aux conditions météorologiques et aux vents peuvent être prises en compte pour affiner la détermination de fraction volumique.

La fraction volumique déterminée peut être comparée à une base de données de fractions volumiques prédéfinies.

Comme susmentionné, les propriétés fluidiques de la fraction volumique déterminée $\varphi$ sont mises en correspondance avec les propriétés fluidiques prédéterminées de la fraction la plus proche parmi les fractions volumiques prédéfinies.

Selon une étape 708, à partir les propriétés fluidiques PF identifiées pour le profil de houle P, il est vérifié si le profil de houle a des propriétés fluidiques qui correspondent ou non aux propriétés fluidiques d'une surface marine comportant des éléments parasites EP pour la centrale CT (PF⇔EP?).

Lorsque les propriétés fluidiques correspondent à une nappe flottante comportant des espèces flottantes (flèche O en sortie de l'étape 708), alors une alerte est émise selon l'étape 710 (ALERT), par exemple à destination du dispositif distant 110 mentionné plus haut.

Cette alerte permet de prévenir d'un risque d'encombrement de tambour de filtration par exemple. Ainsi, il est possible de mettre en place des mesures préventives pour éviter un colmatage.

Sinon, selon la flèche N en sortie de l'étape 708, les propriétés fluidiques ne correspondent pas à une nappe flottante, ou tout au moins, pas à une nappe flottante menaçante pour la centrale CT. Alors le procédé se termine selon l'étape 712 (END) et peut être réitéré pour réaliser de nouvelles mesures et une nouvelle vérification de présence d'une nappe flottante menaçante dans la zone de surveillance.

En outre, afin de pallier au caractère discontinu dans le temps des informations fournies par les satellites (dépendant de la périodicité des survols au-dessus de la zone d'intérêt), il peut être prévu de coupler la détection de nappe d'espèces marines à des modèles hydrodynamiques numériques. Le principe consiste à injecter dans les modèles hydrodynamiques les nappes flottantes détectées et de modéliser sur les jours suivants la dérive de ces nappes en fonction des courants de marées. Les prévisions météorologiques peuvent aussi être prises en compte pour intégrer notamment l'influence des vents sur les courants marins.

Cette technique permet d'estimer le déplacement à venir du profil de houle correspondant à une nappe flottante menaçante pour une centrale nucléaire, permettant d'anticiper les mesures à mettre en place pour protéger par exemple le circuit de refroidissement de cette centrale.

Par ailleurs, la base de données de fractions volumiques prédéfinies peut être construite à partir :

- d'historique des événements passés de colmatage au niveau de la centrale CT,

- de fraction volumique connue pour des espèces flottantes identifiées,

- de gammes de coefficients d'atténuation ou de viscosité apparentes typiquement observés en fonction des concentrations des bancs d'espèces colmatantes ou autre,

- de comportements propres à des espèces biologiques,

- de caractéristiques de corps flottants en surface libre non miscible,

- ou autre.

La base de données peut se présenter sous la forme d'une table multidimensionnelle qui comprend des fractions volumiques de corps flottants classée par saison de l'année, température de l'eau, etc.

La prise en compte des comportements propres à des espèces biologiques permet notamment d'aider à distinguer des espèces entre elles lors de l'identification des propriétés fluidiques. Par exemple, l'évolution dans la colonne d'eau de certaines espèces de méduses en fonction de la luminosité se traduit par une détectabilité dépendante du moment de la journée (évolution des propriétés fluidiques selon le comportement des espèces flottantes au cours d'une journée).

Cette base de données permet d'affiner la relation empirique entre viscosité apparente et teneur en particules du mélange en suspension, et d'attribuer pour chaque type d'espèces marines (par types d'algues, types de gélatineux...) des coefficients empiriques propres.

On comprend donc que l'invention repose sur le principe que les vagues en mer peuvent être modifiées/atténuées lorsqu'elles rencontrent des bancs d'espèces marines (par exemple : méduses, algues...).

La détection de nappe flottante permet de déclencher une alerte préventive lorsque qu'un ou plusieurs bancs d'espèces marines ou hydrocarbures s'approchent d'une centrale nucléaire (et plus particulièrement des tambours de filtrage du système de refroidissement).

Le procédé et le système de détection proposés ont notamment pour avantage :

-   d'offrir une grande couverture spatiale, et

-   d'être opérationnel de jour comme de nuit, sans contrainte de couverture nuageuse.

Potentiellement, le procédé et le système de détections proposés peuvent aussi permettre la détection de dégazage de navires en pleine mer, notamment de nuit.

L'invention a été décrite en référence à des modes de réalisations particuliers qui ne sont pas limitatifs. Bien entendu, la présente invention ne se limite pas à la forme de réalisation décrite à titre d'exemple et elle s'étend à d'autres variantes. Par exemple, le procédé et le système de détection peuvent également être mis en oeuvre pour des installations telles que des fermes éoliennes off-shore pour s'assurer de leur accessibilité lors des opérations de maintenance.

**Revendications**

**1.**   Procédé de détection de nappe flottante (NF) sur une zone de surveillance (ZS) de surface marine, un site d'intérêt (CT) étant placé dans ou en périphérie de la zone de surveillance (ZS), le procédé comprenant au moins les étapes de :

a) mesure satellitaire d'un retour de signal radar, le signal radar étant émis par un satellite vers la surface marine de la zone de surveillance (ZS) ;
b) reconnaissance d'au moins un profil de houle de la surface marine en fonction des mesures satellitaires ;

c) identification des propriétés fluidiques correspondant aux profils de houle reconnus ; et
d) émission d'une alerte lorsque les propriétés fluidiques identifiées pour un des profils reconnus correspondent à une surface marine comportant des éléments parasites pour le site d'intérêt (CT) ;

dans lequel à l'étape b), des limites singulières (LS) de profil de houle sont en outre reconnues sur la zone de surveillance (ZS), les limites singulières (LS) correspondant à de fortes variations localisées des niveaux de mesures satellitaires ;
l'au moins un profil de houle reconnu correspondant à un ensemble de mesures dont le niveau varie selon une même longueur d'onde du profil de houle reconnu et une même amplitude, l'ensemble de mesures étant délimité au moins en partie par les limites singulières (LS) reconnues ;
**caractérisé en ce que**
à l'étape c), les propriétés fluidiques de l'au moins un profil de houle reconnu sont identifiées par :

-   détermination d'une fraction volumique en fonction de :

○ un coefficient d'atténuation de l'amplitude de la houle au niveau des limites singulières (LS),
○ un nombre d'ondes au niveau des limites singulières (LS) déterminé à partir de la longueur d'onde du profil de houle reconnu,

-   identification de la fraction la plus proche de la fraction volumique déterminée parmi des fractions volumiques prédéfinies, les fractions volumiques prédéfinies étant associées à des propriétés fluidiques prédéterminées ; et
-   association au profil de houle reconnu des propriétés fluidiques prédéterminées de la fraction volumique prédéfinie identifiée comme la plus proche de la fraction volumique déterminée.

**2.**   Procédé selon la revendication 1, dans lequel la zone de surveillance (ZS) est dimensionnée en fonction de paramètres parmi :

-   des vitesses de courants marins ;
-   un coefficient de marée ;
-   des conditions météorologiques ;
-   une fréquence de surveillance prédéterminée ; et
-   une fréquence de survol de la zone de surveillance (ZS) par le satellite.

**3.**   Procédé selon l'une des revendications précédentes, dans lequel l'au moins un profil de houle reconnu correspond à un ensemble de mesures sensiblement de même niveau, l'ensemble de mesures étant délimité au moins en partie par les limites singulières reconnues.

4. Procédé selon la revendication 3, dans lequel est reconnu sur la zone de surveillance (ZS) :

- un premier profil de houle selon un ensemble de mesures (P1) sensiblement d'un même premier niveau (N1),
- un deuxième profil de houle selon un ensemble de mesures (P2) sensiblement d'un même deuxième niveau (N2),

dans lequel les premier et deuxième niveaux sont comparés, et
dans lequel le premier ou le deuxième profil de houle correspondant au plus faible des niveaux comparés est associé à des propriétés fluidiques de surface marine comportant des éléments parasites pour le site d'intérêt (CT).

5. Procédé selon l'une des revendications précédentes, dans lequel la fraction volumique est en outre fonction d'une tension superficielle de la surface marine correspondant au profil de houle reconnu.

6. Procédé selon l'une des revendications précédentes, dans lequel les fractions volumiques prédéfinies correspondent à des fractions volumiques constatées, dans des conditions météorologiques connues, pour des surfaces maritimes comportant des corps flottants identifiés.

7. Procédé selon l'une des revendications précédentes, dans lequel les limites singulières (LS) reconnues sont comparées à une cartographie bathymétrique relative à la zone de surveillance (ZS).

8. Procédé selon l'une des revendications précédentes, comprenant en outre une étape de détermination de déplacement du profil de houle correspondant à une surface marine comportant des éléments parasites pour le site d'intérêt (CT), la détermination de déplacement étant fonction de modèles hydrodynamiques préétablis de la zone de surveillance (ZS) ou de modèles hydrodynamiques issus de sources d'information sur les conditions hydrodynamiques de la zone de surveillance (ZS).

9. Système de détection (S) de nappe flottante (NF) sur une zone de surveillance (ZS) de surface marine, un site d'intérêt (CT) étant placé dans ou en périphérie de la zone de surveillance (ZS), le système comprenant au moins :

- un satellite configuré pour :

◦ émettre un signal radar vers la surface marine de la zone de surveillance (ZS) ;
◦ mesurer un retour du signal radar émis ;
◦ communiquer des données relatives aux niveaux de mesure des retours de signal mesurés ;

- une unité de gestion comportant :

◦ une interface de communication configurée pour recevoir les données communiquées par le satellite, et
◦ une unité de traitement de données configurée pour mettre en oeuvre les étapes du procédé de détection selon l'une des revendications 1 à 8.

10. Système de détection (S) selon la revendication 9, comprenant en outre une base de données configurée pour stocker des données relatives à :

- des fractions volumiques prédéfinies correspondant à des fractions volumiques constatées, dans des conditions météorologiques connues, pour des surfaces maritimes comportant des corps flottants identifiés ; et/ou
- une cartographie bathymétrique relative à la zone de surveillance (ZS).

11. Système de détection (S) selon l'une quelconque des revendications 9 et 10, dans lequel le site d'intérêt (CT) est une installation de production électrique.

12. Produit programme informatique comportant des instructions pour la mise en oeuvre du procédé selon l'une des revendications 1 à 8, lorsque ce programme est exécuté par une unité de traitement de données telle qu'un processeur.

**Patentansprüche**

1. Verfahren zur Detektion einer Schwimmschicht (NF) auf einer Meeresoberfläche-Überwachungszone (ZS), wobei ein Ort von Interesse (CT) sich innerhalb oder in der Umgebung der Überwachungszone (ZS) befindet, wobei das Verfahren mindestens die folgenden Schritte aufweist:

a) Satellitenmessung eines Radar-Rücklaufsignals, wobei das Radarsignal von einem Stalliten hin zur Meeresoberfläche der Überwachungszone (ZS) ausgesendet worden ist;
b) Erkennung mindestens eines Dünungsprofils der Meeresoberfläche als Funktion der Satellitenmessungen;
c) Identifizierung von Fluideigenschaften, die mit den erkannten Dünungsprofilen in Beziehung stehen; und

d) Aussendung eines Alarms, wenn die für eines der erkannten Profile identifizierten Fluideigenschaften einer Meeresoberfläche entsprechen, die Elemente aufweist, die für den Ort von Interesse (CT) schädlich sind;

in welchem im Schritt b) ferner singuläre Grenzen (LS) des Dünungsprofils auf der Überwachungszone (ZS) erkannt werden, wobei die singulären Grenzen (LS) starken lokalen Variationen der Satellitenhöhenmessungen entsprechen;

das mindestens eine erkannte Dünungsprofil einer Gruppe von Messwerten entspricht, deren Höhe mit einer gleichen Wellenlänge des erkannten Dünungsprofils und gleicher Amplitude variiert, wobei die Gruppe von Messwerten mindestens teilweise von den erkannten singulären Grenzen (LS) begrenzt wird,

**dadurch gekennzeichnet, dass**

im Schritt c) die Fluideigenschaften des mindestens einen erkannten Dünungsprofils identifiziert werden durch:

- Ermittlung eines Volumenanteils als Funktion

\* eines Dämpfungskoeffizienten der Amplitude der Dünung im Bereich der singulären Grenzen (LS),
\* einer aus der Wellenlänge des erkannten Dünungsprofils ermittelten Wellenzahl im Bereich der singulären Grenzen (LS),

- Identifizierung eines Anteils, die dem ermittelten Volumenanteil am nächsten ist, aus vorab definierten Volumenanteilen, wobei die vorab definierten Volumenanteile mit vorab ermittelten Fluideigenschaften verknüpft sind; und
- Verknüpfung des erkannten Dünungsprofils mit vorab ermittelten Fluideigenschaften des vorab definierten Volumenanteils, die identifiziert wurde als dem ermittelten Volumenanteil am nächsten zu sein.

2. Verfahren nach Anspruch 1, in welchem die Überwachungszone (ZS) als Funktion von Parametern dimensioniert wird, zu denen zählen:

- Geschwindigkeiten von Meeresströmungen;
- ein Gezeitenkoeffizient;
- meteorologische Bedingungen;
- eine vorgegebene Überwachungsfrequenz; und
- eine Überflugfrequenz, mit welcher die Überwachungszone (ZS) von dem Satelliten überflogen wird.

3. Verfahren nach einem der vorstehenden Ansprüche, in welchem das mindestens eine erkannte Dünungsprofil einer Gruppe von Messwerten einer im Wesentlichen gleichen Höhe entspricht, wobei die Gruppe von Messwerten mindestens teilweise von den erkannten singulären Grenzen begrenzt wird.

4. Verfahren nach Anspruch 3, in welchem auf der Überwachungszone (ZS) erkannt wird:

- ein erstes Dünungsprofil gemäß einer Gruppe von Messwerten (P1), die im Wesentlichen auf einer gleichen ersten Höhe (N1) sind,
- ein zweites Dünungsprofil gemäß einer Gruppe von Messwerten (P2), die im Wesentlichen auf einer gleichen zweiten Höhe (N2) sind,

in welchem die erste und zweite Höhe verglichen werden, und

in welchem das erste oder das zweite Dünungsprofil, je nachdem, welches die geringste der verglichenen Höhen hat, verknüpft wird mit Fluideigenschaften einer Meeresoberfläche, die für den Ort von Interesse (CT) schädliche Elemente aufweist.

5. Verfahren nach einem der vorstehenden Ansprüche, in welchem der Volumenanteil ferner eine Funktion einer Oberflächenspannung der dem erkannten Dünungsprofil entsprechenden Meeresoberfläche ist.

6. Verfahren nach einem der vorstehenden Ansprüche, in welchem die vorab definierten Volumenanteile Volumenanteilen entsprechen, die für Seeoberflächen, die identifizierte Schwimmkörper aufweisen, unter bekannten meteorologischen Bedingungen ermittelt wurden.

7. Verfahren nach einem der vorstehenden Ansprüche, in welchem die erkannten singulären Grenzen (LS) mit einer die Überwachungszone (ZS) betreffenden bathymetrischen Karte verglichen werden.

8. Verfahren nach einem der vorstehenden Ansprüche, aufweisend ferner einen Schritt einer Ermittlung einer Verlagerung des Dünungsprofils, das einer Meeresoberfläche entspricht, die für den Ort von Interesse (CT) schädliche Elemente aufweist, wobei die Ermittlung der Verlagerung abhängig von vorab aufgestellten hydrodynamischen Modellen der Überwachungszone (ZS) oder auf hydrodynamischen Modellen, die aus Informationsquellen über die hydrodynamischen Bedingungen der Überwachungszone stammen, ist.

9. Detektionssystem (S) zur Detektion einer Schwimmschicht (NF) auf einer Meeresoberfläche-Überwachungszone (ZS), wobei ein Ort von Interesse (CT) sich innerhalb oder in der Umgebung der Überwachungszone befindet, wobei das System mindestens aufweist:

- einen Satelliten, der konfiguriert ist, um

* ein Radarsignal hin zur Meeresoberfläche der Überwachungszone (ZS) auszusenden;
* einen Rücklauf des ausgesendeten Radarsignals zu messen;
* Messhöhen betreffende Daten der gemessenen Signalrückläufe zu übermitteln;

- eine Steuereinheit, die aufweist:

* eine Kommunikationsschnittstelle zum Empfangen der von dem Satelliten übermittelten Daten und
* eine Datenverarbeitungseinheit zum Durchführen der Schritte des Detektionsverfahrens nach einem der Ansprüche 1 bis 8.

10. Detektionssystem (S) nach Anspruch 9, ferner aufweisend eine Datenbank zum Speichern der Daten, die betreffen

- vorab definierte Volumenanteile, die Volumenanteilen entsprechen, die für Seeoberflächen, die identifizierte Schwimmkörper aufweisen, unter bekannten meteorologischen Bedingungen ermittelt wurden; und/oder
- eine die Überwachungszone (ZS) betreffende bathymetrische Karte.

11. Detektionssystem (S) nach einem der Ansprüche 9 und 10, in welchem der Ort von Interesse (CT) eine Anlage zur Stromerzeugung ist.

12. Informatikprogrammprodukt aufweisend Befehle zur Durchführung des Verfahrens nach einem der Ansprüche 1 bis 8, wenn dieses Programmprodukt auf einer Datenverarbeitungseinheit, beispielsweise einem Prozessor, ausgeführt wird.

**Claims**

1. A method for detecting a floating layer (NF) on a surveillance area (ZS) of a sea surface, a site of interest (CT) being located in or at a rim of the surveillance area (ZS), the method comprising at least the steps of:

a) satellite measurement of a returned radar signal, the radar signal being transmitted by a satellite toward the sea surface of the surveillance area (ZS);

b) recognition of at least one swell profile of the sea surface, based on the satellite measurements;
c) identification of fluid properties corresponding to the recognized swell profiles; and
d) emission of a warning when the fluid properties identified for one of the recognized profiles correspond to a sea surface comprising elements that are undesirable for the site of interest (CT);

wherein, in step b), distinctive boundaries (LS) of swell profile are further recognized in the surveillance area (ZS), the distinctive boundaries (LS) corresponding to pronounced localized variations in levels of the satellite measurements;
the at least one recognized swell profile corresponding to a set of measurements whose level varies according to a same wavelength of the recognized swell profile and a same amplitude, the set of measurements being delimited at least in part by the recognized distinctive boundaries (LS);
**characterized in that**:
in c), the fluid properties of the at least one recognized swell profile are identified by:

- determining a volume fraction as a function of:

∘ a damping coefficient of amplitude of the swell at the distinctive boundaries (LS),
∘ a number of waves at the distinctive boundaries (LS), determined from the wavelength of the recognized swell profile,

- identifying the fraction among the predefined volume fractions that is closest to the determined volume fraction, the predefined volume fractions being associated with predetermined fluid properties; and
- associating the recognized swell profile with the predetermined fluid properties of the predefined volume fraction identified as being closest to the determined volume fraction.

2. The method according to claim 1, wherein dimensions of the surveillance area (ZS) are based on at least one parameter among:

- speeds of the ocean currents;
- a tidal coefficient;
- weather conditions;
- a predetermined surveillance frequency; and
- frequency at which the satellite passes over the surveillance area (ZS).

3. The method according to one of the preceding claims, wherein the at least one recognized swell profile corresponds to a set of measurements of substantially a same level, the set of measurements being bounded at least in part by the recognized distinctive boundaries.

4. The method according to claim 3, wherein, in the surveillance area (ZS):

   - a first swell profile is recognized from a set of measurements (P1) of substantially a same first level (N1),
   - a second swell profile is recognized from a set of measurements (P2) of substantially a same second level (N2),

   wherein the first level and the second level are compared, and
   wherein the swell profile corresponding to a lower of the compared levels among the first swell profile and the second swell profile is associated with the fluid properties of a sea surface having elements that are undesirable for the site of interest (CT).

5. The method according to one of the preceding claims, wherein the volume fraction further is function of a surface tension of the sea surface corresponding to the recognized swell profile.

6. The method according to one of the preceding claims, wherein the predefined volume fractions correspond to volume fractions observed, in known weather conditions, for sea surfaces having identified floating bodies.

7. The method according to one of the preceding claims, wherein the recognized distinctive boundaries (LS) are compared to a bathymetric mapping of the surveillance area (ZS).

8. The method according to one of the preceding claims, further comprising a step of determining movement of the swell profile corresponding to a sea surface comprising elements that are undesirable for the site of interest (CT), the determining of the movement being based on pre-established hydrodynamic models of the surveillance area (ZS) or on hydrodynamic models from sources of information about the hydrodynamic conditions of the surveillance area (ZS).

9. A detection system (S) for detecting a floating layer (NF) in a surveillance area (ZS) of a sea surface, a site of interest (CT) being located in or at a rim of the surveillance area (ZS), the system comprising at least:

   - a satellite configured to:

     ∘ transmit a radar signal toward the sea surface of the surveillance area (ZS);
     ∘ measure a return signal of the transmitted radar signal;
     ∘ communicate data concerning the measurement levels of the measured return signals;

   - a management unit comprising:

     ∘ a communication interface configured to receive data communicated by the satellite, and
     ∘ a data processing unit configured to implement the steps of the detecting method of any one of claims 1 to 8.

10. The detection system (S) according to claim 9, further comprising a database configured for storing data relating to:

    - predefined volume fractions corresponding to volume fractions observed, in known weather conditions, for sea surfaces having identified floating bodies; and/or
    - a bathymetric mapping of the surveillance area (ZS).

11. The detection system (S) according to any one of claims 9 and 10, wherein the site of interest (CT) is a power generation facility.

12. A computer program product comprising instructions for implementing the method according to one of claims 1 to 8, when the program is executed by a data processing unit such a processor.

FIG. 1a

**FIG. 1b**

FIG. 2

FIG. 3

FIG. 4

FIG. 5

**FIG. 6**

700 — DEF (ZS)

702 — MES

704 — REC (P)

706 — ID (PF)

708 — PF ⟺ EP ?

**FIG. 7**

O

N

710 — ALERT

712 — END

## RÉFÉRENCES CITÉES DANS LA DESCRIPTION

**Littérature non-brevet citée dans la description**

- **MASAHARU FUJITA et al.** SIR-B Experiments in Japan: Sensor Calibration and Oil Pollution Détection Over Océan. *IEEE Transactions on Geoscience and Remote Sensing,* 01 Juillet 1986, vol. 44, 567-574
- **PETER VOGT et al.** Monitoring of marine oil spills from SAR satellite data. *Proceedings of SPIE,* 16 Novembre 2004, vol. 5569, 209-219
- Application of Ground-Based And Air/Spaceorne Radars for Oil Spill Détection in Sea Areas. **E. N. BELOV et al.** Télécommunications and Radio Engineering. Scripta Technica Inc, 01 Janvier 1997, vol. 51, 1-08
- **YAHYA A. DEHQANZADA et al.** *Secrets For Sale: How Commercial Satellite Imagery Will Change the World,* 01 Janvier 2000, http:// carnegieendowment.org/files/FINALreport.pdf
- **MERV FINGAS et al.** Review of oil spill remote sensing. *Marine Pollution Bulletin,* 20 Avril 2014, vol. 83, 9-23